# DEMANDE DE BREVET EUROPEEN

(11) **EP 1 658 826 A1**
(43) Date de publication de la demande: **24.05.2006**
(21) Numéro de dépôt: 04447258.7
(22) Date de dépôt: 23.11.2004
(51) Int. Cl.: A61C 13/087, A61K 6/083

(54) **Composition pour une dent artificielle**

(71) Demandeur: Simonis Plastic, 4430 Ans (BE)
(72) Inventeur: Verhoyen, Olivier, 4280 Hannut (BE); Melot, Charles, 5030 Gembloux (BE)
(74) Mandataire: Van Malderen, Michel

(57) **Abrégé**

La présente invention se rapporte à une dent artificielle ou à un endobloc renforcés, comportant au moins une partie renforcée, ladite partie renforcée comportant une matrice polymérique thermoplastique ainsi qu'une charge minérale choisie parmi un ou plusieurs éléments du groupe des microsphères creuses et des microbilles pleines en céramique ou en verre

## Description

### Objet de l'invention

La présente invention concerne des dents artificielles et des endoblocs en matière synthétique utilisés comme matériel d'expérimentation endodontique à des fins de perfectionnement ou d'apprentissage. Elles servent également à des essais de matériel de traitement dentaire.

### Arrière-plan technologique

En vue de parfaire la formation de futurs dentistes, on utilisait autrefois pour l'apprentissage des techniques de dentisterie, des mâchoires munies de leurs dents naturelles, qui étaient notamment prélevées sur des cadavres.

Pour des raisons évidentes d'hygiène et dans un souci de standardisation de l'évaluation des étudiants, on a proposé de travailler sur des mâchoires comportant des dents artificielles imitant le mieux possible les dents naturelles.

Une utilisation supplémentaire de dents artificielles est de tester ou de faire la démonstration de nouvelles techniques de soin et/ou de nouveaux dispositifs, c'est la raison pour laquelle la similitude de comportement des outils de dentisterie sur les dents naturelles et les dents artificielles est importante.

La difficulté principale réside dans le fait que la plupart des dents artificielles ou endoblocs ne procurent pas aux étudiants les sensations qu'ils éprouvent en travaillant sur des dents naturelles. La réalisation d'une « dentine artificielle », reproduisant exactement les mêmes sensations qu'une dentine naturelle répond donc à une attente de longue date des enseignants en dentisterie.

### Etat de la technique

Le document EP 1045363 A1, déposé par l'inventeur, divulgue une dent artificielle comportant une matrice polymérique thermoplastique et son procédé de fabrication. Les polymères utilisés dans ce document pour la racine sont le polyméthacrylate de méthyle et le polycarbonate. Les polymères utilisés pour la couronne sont le PPS ou le PAA. La matrice polymérique peut contenir des charges minérales telles que des fibres de verre.

Le document DE 19534505 décrit une dent artificielle destinée à une reconstitution de la couronne ayant une couche externe comportant un polymère thermodurcissable contenant une charge inorganique, telle que le silicate et l'oxyde d'aluminium. L'utilisation d'un polymère thermodurcissable, comporte cependant une série de difficultés techniques liées notamment à la réticulation de ces polymères ce qui ne permet qu'un choix restreint de charges minérales.

Le document GB 1143502 décrit une composition pour dents artificielles constituées d'une résine PMMA thermodurcissable, d'un pigment et d'une charge inorganique comme la fibre de verre. Ces dents artificielles sont formées par moulage. Il s'agit d'une composition à base de PMMA broyé en poudre et d'acrylate réticulable et non d'un PMMA thermoplastique. Les charges sont essentiellement utilisées pour opacifier la composition. La composition développée est utilisée également comme enrobage extérieur d'un coeur en PMMA pur.

### Eléments caractéristiques de l'invention

La présente invention divulgue une dent artificielle ou un endobloc renforcés, comportant au moins une partie renforcée, ladite partie renforcée comportant une matrice polymérique thermoplastique ainsi qu'une charge minérale choisie parmi un ou plusieurs éléments du groupe des microsphères creuses et des microbilles pleines en céramique ou en verre.

Selon des formes particulières de réalisation, l'invention comporte par ailleurs l'une ou plusieurs des caractéristiques suivantes :
- la matrice polymérique de la partie renforcée est choisie parmi un ou plusieurs polymères du groupe du PMMA, PS, PC, SPS, SAN et COC ;
- ladite céramique utilisée pour les microbilles ou les microsphères est une céramique à base silicate d'alumine alcalin ;
- le verre utilisé pour les microbilles ou les microsphères est un verre à base de borosilicate à chaux sodée ;
- la charge minérale de la partie renforcée comporte en outre du sulfate de baryum ;
- la partie renforcée comporte une charge minérale représentant entre 10 et 50% en poids par rapport à la résine ;
- la matrice polymérique de la partie renforcée comporte du PMMA avec un indice de fluidité compris entre 5 et 20g/10 min mesuré à 230°C/3,8 Kg (ISO 1133) ;
- les microbilles de verre ont un diamètre inférieur à 100µm, de préférence de 10-50µm ;
- les microsphères en verre ont un diamètre de 30-80µm ;
- les microsphères en céramique ont un diamètre de 5-25µm ;
- la dent artificielle ou endobloc comporte une partie transparente et une partie non transparente, la partie non transparente étant la partie renforcée.

### Description des figures

La figure 1 représente une dent artificielle en plusieurs parties qui est un mode préféré de réalisation de l'invention.

La figure 2 représente un endobloc en deux parties correspondant également à une forme d'exécution préférée de l'invention.

La figure 3 représente une réalisation selon l'invention avec une couronne en PPS renforcée de fibres de verre et de sphères de verre selon l'invention et une racine transparente en PMMA non renforcée.

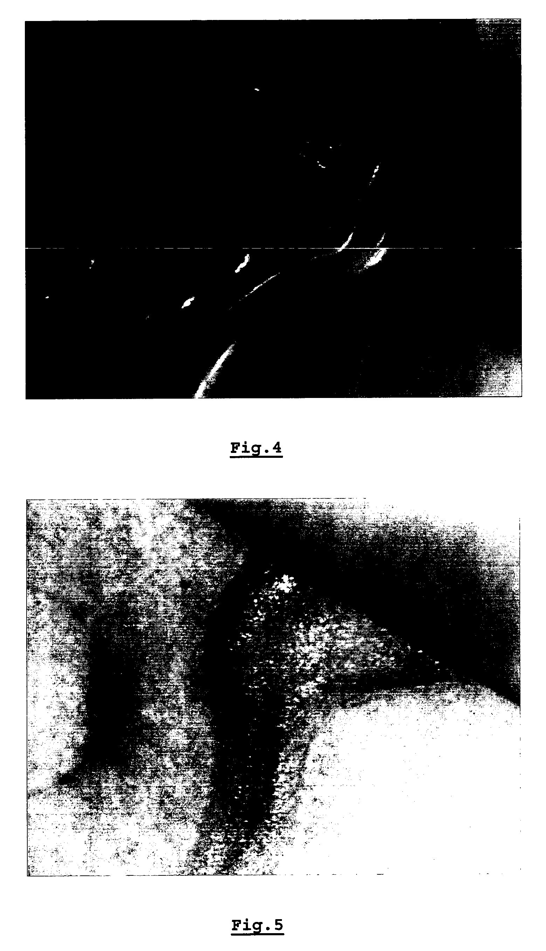

La figure 4 représente une vue de détail d'un endobloc en PMMA non chargé et en particulier l'aspect de la surface à l'entrée du canal du nerf (grossissement 50x).

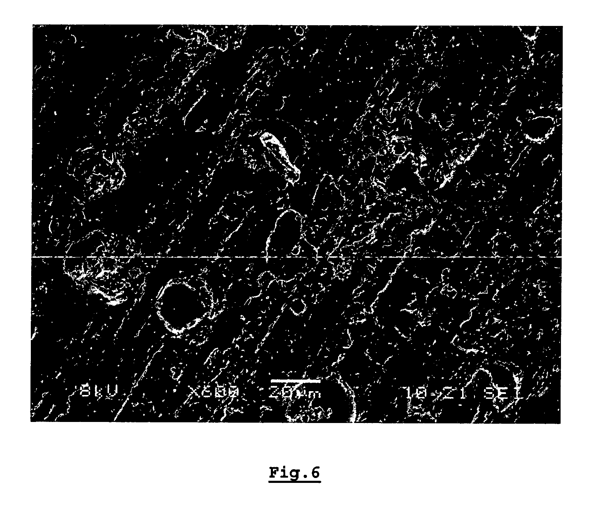

La figure 5 représente une vue de détail d'un endobloc en PMMA chargé à 25% de microsphères de verre⁴ et en particulier l'aspect de la surface à l'entrée du canal du nerf (grossissement 50x).

La figure 6 montre une vue au microscope électronique d'une coupe de la matrice polymérique selon la présente invention comportant des microsphères.

### Description de l'invention

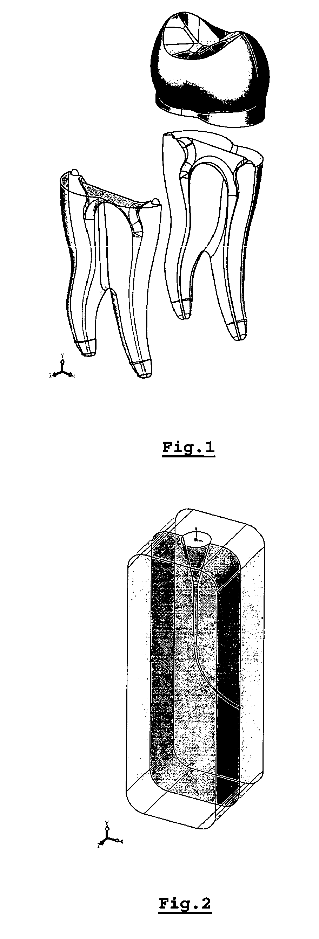

Les dents synthétiques selon la présente invention, reproduisent fidèlement la morphologie interne d'une dent naturelle. A ce titre elles peuvent comporter une corne pulpaire, une chambre pulpaire, une taille et une géométrie variable des canaux comme par exemple un canal en C, commun dans la réalité et un canal en S, simulant une complication réelle. (voir figure 1).

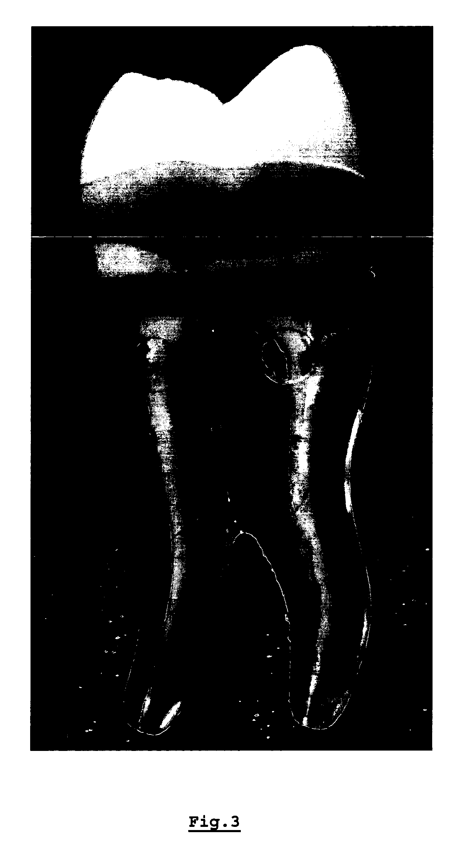

Les différentes parties de la dent peuvent êtres transparents ou opaques, elles peuvent également se présenter sous forme semi-transparente ou mixte, c'est à dire avec une partie transparente et une partie opaque tel que présenté dans la figure 3.

Dans le cas ou seul le traitement canalaire doit être étudié, une version simplifiée de la dent peut être réalisée au moyen des mêmes matériaux, celle-ci est appelée endobloc. Un tel endobloc est constitué de deux parallélépipédiques enfermant un canal. Selon le cas, il pourra se décliner avec ou sans ergot dentaire.

La dent artificielle et l'endobloc selon l'invention sont avantageusement réalisés dans des matériaux dont les caractéristiques mécaniques minimales doivent rencontrer au moins les exigences suivantes pour que les conditions rencontrées dans la réalité soient respectées et que les sensations réelles, satisfaisant les spécialistes en endodontie, soient reproduites :
- possibilité d'usinage de la dent avec une fraise tournant à 50000 tours/minute,
- possibilité d'usinage des canaux radiculaires manuellement ou à l'aide d'une fraise mécanique tournant à 300 tours/minute,
- possibilité d'obturation des canaux radiculaires alésés avec un liant dont la température peut atteindre 85°C et qui est appliqué par projection à l'aide d'un outil tournant à 5000 tours/minute,
- apparition de copeaux comme pour la dentine,
- progression manuelle ou en rotation continue,
- possibilité d'usinage de la dent avec ou sans lubrification,
- possibilité de retraitement endodontique.

Comme nous l'avons dit plus haut, les dents artificielles et les endoblocs sont parfois réalisés en plusieurs parties pouvant comporter des parties transparentes (parties de racines ou de couronnes), à des fins didactiques. Selon l'invention, au moins une partie de la dent artificielle ou de l'endobloc est renforcée et est susceptible d'avoir le même comportement au traitement mécanique que la dentine naturelle.

Pour élaborer cette partie renforcée de la dent artificielle ou de l'endobloc, les inventeurs ont procédé à l'ajout de charges minérales d'une granulométrie spécifique.

Le pourcentage en charge minérale ne devrait pas dépasser les 50% en poids par rapport à la résine. Au-delà de ce pourcentage le travail de la matière plastique s'avère difficile et le matériau devient cassant.

Parmi les charges minérales les plus efficaces, on trouve des microsphères en céramique ou en verre. Ce sont des corps creux. On peut également utiliser des microbilles en verre, du BaSO₄, du talc, du CaCO₃ ou encore des fibres de verre. Le diamètre et la forme (creuse ou non) de la charge minérale ont une influence importante sur les caractéristiques mécaniques de la dent artificielle et sur l'authenticité des sensations procurées aux étudiants lors des exercices pratiques dont il a été question plus haut.

Les microbilles de verre et de céramique utilisées ont une forme sphérique ou sphéroïde. Ces billes peuvent être creuses (glass bubbles-scotchlite™ 3M), dans ce cas on parlera de microsphères, ou pleines, dans ce cas on parlera de microbilles (Zeeospheres™ 3M - céramique).

La définition de microsphères selon la présente invention est à prendre au sens large, elle englobe également des particules de verre creuses de forme sphéroïde et multicellulaires du type Noblite®. Ces « bulles » de verre sont obtenues à partir de Rhyolite qui est une roche volcanique d'une composition proche de celle du granite (quartz, feldspath, mica).

Les verres sont par exemple des (boro)silicates à chaux sodée de composition suivante :
- SiO₂: 70 à 75%
- CaO: 7 à 11%
- Na₂O: 13 à 15%
- Al₂O₃: < 2%
- MgO: < 5%
- K₂O: < 1%
La densité réelle de ce type de billes est de 2,46 Kg/dm3 et la densité apparente de 1,6 Kg/dm3.
La densité des sphères de verre est environ de 0,6 Kg/dm3 et la densité apparente est environ de 0,4 Kg/dm3.
Les céramiques utilisées sont par exemple du type silicate d'aluminium alcalin.

Les microbilles ou microsphères peuvent être pourvues en surface d'un agent de couplage permettant un lien intime entre la matrice polymérique et les billes/sphères. Les billes/sphères, ont une surface spécifique très faible par rapport aux poudres ayant une granulométrie analogue.

Le pourcentage en poids de charge minérale doit se situer idéalement entre 10 et 50% en poids par rapport au poids total de la composition renforcée, il doit se situer de préférence entre 15 et 40% et de manière particulièrement préférée entre 20 et 30% pour obtenir un résultat optimal. Le résultat est cependant fonction à la fois du comportement de la matrice polymérique en tant que telle, mais également de la nature de la charge. Les microbilles pleines ayant une densité environ quatre fois plus élevée que les microsphères creuses, leur pourcentage en poids peut être nettement supérieur tout en occupant un pourcentage en volume identique. C'est ainsi qu'un pourcentage en poids de microsphères creuses de 50% en poids sera difficilement atteint car à ce pourcentage, les microsphères creuses occupent pratiquement l'ensemble du volume théorique de la partie renforcée (chargée). Pour les microbilles pleines, ce pourcentage ne présente cependant pas de difficultés particulières.

Le mélange des polymères et des charges minérales se fait par des procédés bien connus des plasturgistes. Dans le cas particulier du PMMA le compoundage a été effectué sur une extrudeuse MAPRE avec deux vis corotatives. Le PMMA, après avoir été préalablement séché à 80°C est porté à une température entre 200 et 220°C dans l'extrudeuse tout en étant mélangé aux charges minérales. Le mélange homogène est ensuite extrudé à travers une filière de 5mm et granulé de manière classique. Ces granulés sont ensuite utilisés pour le procédé d'injection de la dent artificielle ou de l'endobloc, ou du moins de leur partie renforcée.

La procédure qui vient d'être décrite est bien entendu analogue pour tous les polymères thermoplastiques.

Dans le cas de l'utilisation de microsphères de verre par exemple, une charge minérale d'environ 25% en poids par rapport au poids total de la composition, fournit un résultat optimal, alors que dans le cas de l'utilisation de microsphères céramiques, 40% en poids sont nécessaires pour reproduire les caractéristiques d'instrumentation de la dentine naturelle.

Des combinaisons de charges minérales entre elles sont également possibles, par exemple en mélangent les charges minérales citées plus haut à un pourcentage minimum de sphères de verre pour obtenir un comportement similaire. Néanmoins, il est préférable que 20 à 25% de microsphères de verre soit présentes pour reproduire une sensation authentique lors de l'instrumentation de la racine. Pour les raisons évoquées plus haut, le pourcentage de microbilles pourra être moins élevé que 20% en poids pour aboutir au même effet.

Le mélange des charges minérales avec du sulfate de baryum donne une radio opacité qui permet de vérifier à postériorité le travail réalisé par les étudiants en effectuant une radio des dents instrumentées. Le sulfate de baryum est bien connu pour absorber le rayonnement X alors que la plupart des autres charges sont radiotransparentes.

La dent artificielle est généralement assemblée à partir de différents constituants, pouvant avoir des compositions différentes.

La composition de la couronne peut être différente de celle de la racine. La couronne peut par exemple être constituée de PPS + 40% de fibres de verre + 20 % charges minérales de microbilles/microsphères.

Les inventeurs ont démontré que l'utilisation de billes de verre d'un diamètre supérieur à 100*µ*m (Test 2) ne reproduisait pas une dentine artificielle authentique. Le diamètre optimal pour les billes de verre se situe entre 5-25*µ*m. Le diamètre optimal des sphères se situe entre 30-80*µ*m.

L'utilisation d'une microsphère (charge creuse) donne les meilleurs résultats car le matériau présente un comportement à la coupe identique à la dentine naturelle. Il a également été remarqué lors de l'instrumentation des canaux que les microsphères, en contact direct avec le canal du nerf, étaient détruites par l'outil et formaient de ce fait des creux le long du canal du nerf. Ces creux peuvent être apparentés aux tubulis présents dans les dents naturelles.

Les exemples 1 à 15 déterminent le type de charges minérales les plus efficaces de la présente invention en utilisant uniquement le PMMA. Le tableau 1 montre que la présence de sphères creuses en verre et de microbilles de céramique et/ou en verre apporte immédiatement un effet bénéfique, ses caractéristiques se rapprochant de la dentine naturelle. L'influence du sulfate de Baryum (BaSO₄,) est également bénéfique. Celui-ci peut remplacer partiellement ou entièrement les sphères ou les billes de verre/céramique.

**Tableau 1**

| **Test** | **Résine** | **Charges minérales** | **Effet** |
|---|---|---|---|
| 1 | PMMA¹ 75% | Billes de verre² 25% | + |
| 2 | PMMA¹ 75% | Billes de verre³ 25% | - |
| 3 | PMMA¹ 75% | Microsphères de verre⁴ 25% | +++ |
| 4 | PMMA¹ 80% | Microsphères de verre⁴ 20% | ++ |
| 5 | PMMA¹ 65% | Microsphères⁴ 20% - BaSO₄ 15% | +++ |
| 6 | PMMA¹ 70% | Microbilles de céramique⁵ 30% | + |
| 7 | PMMA¹ 90% | Microsphères de verre⁴ 10% | - |
| 8 | PMMA¹ 60% | Microsphères de verre⁴ 40% | ++ |
| 9 | PMMA¹ 60% | Microbilles de verre² 40%% | ++ |
| 10 | PMMA¹ 85% | Microbilles de céramique⁵ 15% | - |
| 11 | PMMA¹ 60% | Microbilles de céramique⁵ 50% | ++ |
| 12 | PMMA¹ 50% | Microbilles de céramique⁵ 60% | + |
| 13 | PMMA¹ 70% | Microbilles de céramique⁵ 30% - BaSO₄ 10% | ++ |
| 14 | PMMA¹ 60% | Microsphères de verre⁴ 20% et Microbilles de céramique 20% | +++ |
| 15 | PMMA¹ 70% | Microsphères⁴ 15% - BaSO₄ 15% | ++ |

1. PMMA de la société Lucite Diakon CLG-356 MFI 15g/10min 230°C/3,8Kg.
2. billes de verre 050/20/A0 de la firme Sovitec avec un diamètre moyen de 20µm.
3. billes de verre 50/100/215 de la firme Sovitec avec un diamètre de 100µm.
4. microsphères de verre S60/10000 Scotchlite™ Glass Bubbles de la firme 3M avec un diamètre moyen de 30µm.
5. microbilles céramiques Zeeospheres™ W-210 de la société 3M avec un diamètre de 12µm.

Les essais ont ensuite été étendus à d'autres polymères thermoplastiques également utilisés pour la réalisation au moins une partie des dents artificielles. (SPS,PS,PC,COC,SAN)

**Tableau 2**

| **Test** | **Résine** | **Charges minérales** | **Effet** |
|---|---|---|---|
| 16 | SPS⁶ 77% | Microsphères de verre⁴ 23% | +++ |
| 17 | SPS⁶ 70% | Microbilles de céramiques⁵ 30% | ++ |
| 18 | SPS⁶ 60% | Microsphères de verre⁴ 25% + BaSO₄ 15% | +++ |
| 19 | PS⁷ 75% | Microsphères de verre⁴ 25% | ++ |
| 20 | PS⁷ 75% | Microbilles de céramiques⁵ 25% | + |
| 21 | PS⁷ 80% | Microbilles de céramique⁵ 10% et Microsphères de verre⁴ 10% | - |
| 22 | PC⁸ 70% | Microsphères de verre⁴ 30% | ++ |
| 23 | PC⁸ 80 % | Microbilles de céramique⁵ 20% | + |
| 24 | PC⁸ 80% | Microbilles de céramique⁵ 10% et Microsphères de verre⁴ 10% | ++ |
| 25 | COC⁹ 65% | Microbilles de céramique⁵ 35% | ++ |
| 26 | COC⁹ 75% | Microsphères de verre⁴ 25% | ++ |
| 27 | COC⁹ 60% | Microbilles de céramique⁵ 20% et Microsphères de verre⁴ 20% | +++ |
| 28 | SAN¹⁰ 74% | Microsphères de verre⁴ 26% | ++ |
| 29 | SAN¹⁰ 70% | Microsphères⁴ 15% - BaSO₄ 15% | ++ |
| 30 | SAN¹⁰ 70% | Microbilles de céramique⁵ 20% et Microsphères de verre⁴ 10% | + |

| | | | |
|---|---|---|---|
| 6. SPS Questra WA210 de la société Dow Chemicals | | | |
| 7. PS Empera 123L de la société BP. | | | |
| 8. PC Lexan 144R de la société GE. | | | |
| 9. SAN Luran 388S de la société BASF. | | | |
| 10.COC 8007S-04 de la société Ticona. | | | |

Les polymères du tableau 2 se comportent de la même manière que le PMMA, à quelques nuances près, et peuvent donc également convenir pour la réalisation de l'invention.

## Revendications

1. Dent artificielle ou endobloc renforcé, comportant au moins une partie renforcée, ladite partie renforcée comportant une matrice polymérique thermoplastique ainsi qu'une charge minérale choisie parmi un ou plusieurs éléments du groupe des microsphères creuses et des microbilles pleines en céramique ou en verre.

2. Dent artificielle ou endobloc selon la revendication 1, **caractérisé en ce que** ladite matrice polymérique est choisie parmi un ou plusieurs polymères du groupe du PMMA, PS, PC, SPS, SAN et COC.

3. Dent artificielle ou endobloc selon la revendication 1, **caractérisé en ce que** ladite céramique est une céramique à base silicate d'alumine alcalin.

4. Dent artificielle ou endobloc, selon la revendication 1, **caractérisé en ce que** le verre est un verre à base de borosilicate à chaux sodée.

5. Dent artificielle ou endobloc selon la revendication 1, **caractérisé en ce que** ladite charge minérale comporte en outre du sulfate de baryum.

6. Dent artificielle ou endobloc selon la revendication 1, **caractérisé en ce que** ladite partie renforcée comporte une charge minérale représentant entre 10 et 50% en poids par rapport à la résine.

7. Dent artificielle ou endobloc selon la revendication 1, **caractérisé en ce que** ladite matrice polymérique comporte du PMMA avec un indice de fluidité compris entre 5 et 20g/10 min mesuré à 230°C/3,8 Kg (ISO 1133)

8. Dent artificielle ou endobloc selon la revendication 1, **caractérisé en ce que** les microbilles de verre ont un diamètre inférieur à 100µm.

9. Dent artificielle ou endobloc selon la revendication 8, **caractérisé en ce que** les microbilles de verre ont un diamètre de 10-50µm.

10. Dent artificielle ou endobloc selon la revendication 1, **caractérisé en ce que** les microsphères en verre ont un diamètre de 30-80µm.

11. Dent artificielle ou endobloc selon la revendication 1, **caractérisé en ce que** les microsphères en céramique ont un diamètre de 5-25µm.

12. Dent artificielle ou endobloc selon la revendication 1, comportant une partie transparente et une partie non transparente, la partie non transparente étant la partie renforcée.
